(19) Europäisches Patentamt
European Patent Office
Office européen des brevets

(11) Publication number: **0 125 052**
**B1**

(12) # EUROPEAN PATENT SPECIFICATION

(45) Date of publication of patent specification: **27.01.88**

(51) Int. Cl.⁴: **C 07 D 215/26,**
C 07 D 215/32, A 61 K 31/47
// C07D215/60

(21) Application number: **84302551.1**

(22) Date of filing: **13.04.84**

(54) **Dopaminergic carbostyrils and a process for preparing them.**

(30) Priority: **26.04.83 US 488868**

(43) Date of publication of application:
**14.11.84 Bulletin 84/46**

(45) Publication of the grant of the patent:
**27.01.88 Bulletin 88/04**

(84) Designated Contracting States:
**AT BE CH DE FR GB IT LI LU NL SE**

(56) References cited:
**US-A-4 022 776**

(73) Proprietor: **SMITHKLINE BECKMAN CORPORATION**
**P.O. Box 7929 1 Franklin Plaza**
**Philadelphia Pennsylvania 19101 (US)**

(72) Inventor: **Dandridge, Penelope Ann**
**608 Strath Haven Avenue**
**Swarthmore Pennsylvania 19081 (US)**
Inventor: **Kaiser, Carl**
**1105 Sylvan Drive**
**Haddon Heights New Jersey 08035 (US)**

(74) Representative: **Denerley, Paul Millington, Dr. et al**
**Smith Kline & French Laboratories Ltd**
**Patent Department Mundells**
**Welwyn Garden City Hertfordshire AL7 1EY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 125 052 B1

**Description**

This invention comprises new 5-(2-aminoethyl)-8-hydroxycarbostyril derivatives which have dopaminergic activity and chemical methods for preparing them.

US—A—4 022 776 discloses carbostyril derivatives of the formula (i):

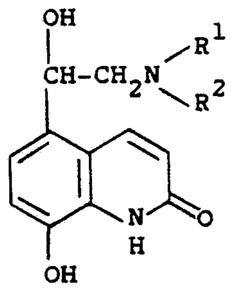

(i)

wherein *inter alia* $R^1$ and $R^2$ each represents a hydrogen atom, a $C_{1-4}$alkyl group, or aralkyl group containing a $C_{1-4}$alkyl moiety. The compounds of formula (i) are disclosed as having β-adreno-receptor stimulating activity.

The present invention comprises a novel group of carbostyril derivatives having a different activity to the compounds of the prior art hereinabove, that is dopaminergic activity.

The compounds of the present invention are illustrated by the following structural formula:

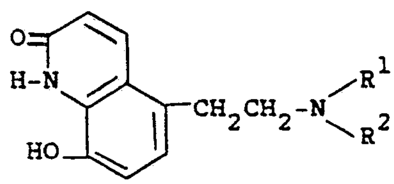

I

in which $R^1$ and $R^2$ are, each, hydrogen, lower alkyl, allyl, benzyl, phenethyl or *p*-hydroxyphenethyl.

A subgeneric group of this invention comprises the compounds of formula I in which —$NR^1R^2$ is amino, di-*n*-propylamino, *n*-propyl-*n*-butylamino or *p*-hydroxyphenethyl-*n*-propylamino.

The term "lower alkyl" is meant to include straight or branched alkyl groups of from 1—6 carbons, preferably *n*-propyl.

The pharmaceutically acceptable, acid addition salts which have the utility of the free bases of formula I are part of this invention. These are prepared by methods well known to the art and are formed with both inorganic or organic acids, for example: maleic, fumaric, benzoic, ascorbic, pamoic, succinic, bismethylenesalicylic, methanesulfonic, ethane disulfonic, acetic, oxalic, propionic, tartaric, salicylic, citric, gluconic, aspartic, stearic, palmitic, itaconic, glycolic, *p*-aminobenzoic glutamic, benzenesulfonic, hydrochloric, hydrobromic, sulfuric, cyclohexylsulfamic, phosphoric and nitric acids. The hydrohalic and, especially, methanesulfonic acid salts are conveniently used.

The reaction sequence which is useful for the chemical preparation of intermediates for preparing the compounds of formula I involves the introduction of a chloromethyl at position 5 of 8-hydroxyquinoline followed by a cyanide exchange with the chloro group, then, reduction of the resulting cyanomethyl compound. After protecting the 8-hydroxy and the primary amino centers, the ring nitrogen atom is oxidized with a mild oxidizing agent such as a perbenzoic acid to give the N-oxide. The latter is converted to a carbostyril by treatment with acetic anhydride.

The various N-alkylated end products of this invention are prepared by stepwise reductive alkylation or mild N-alkylation, the latter using a halide or reactive ester alkylating agent, of the free primary amine. If strong N-alkylating conditions are used, ring N-alkylation is a possible side reaction. The 1-position can be blocked, during such N-alkylation reactions at the side chain, as known to the art.

The compounds of formula I are prepared by reacting a compound of the structural formula:

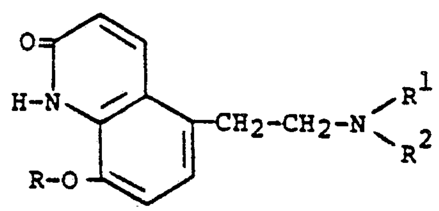

II

in which R is a protecting radical such as lower alkyl or an optionally substituted benzyl and $R^1$ and $R^2$ are as defined above, with deprotecting agent such as an ether cleaving agent, for example, boron tribromide or

trichloride preferably in a halogenated alkane solvent as methylene chloride, carbon tetrachloride or chloroform, 48% aqueous hydrogen bromide, hydriodic acid, pyridine hydrochloride, aluminum chloride or bromide in benzene or carbon disulfide, hydrogen fluoride-antimony pentafluoride, trifluoromethyl sulfonic acid in thioanisole or catalytic hydrogenation over a palladium catalyst when R is a benzyl group.

The splitting reaction is conveniently run using boron tribromide in methylene chloride from 0° to room temperature or 48% hydrogen bromide at reflux temperature.

The compounds of formula I are then isolated by methods known to the art such as neutralizing the reaction mixture and extracting the residual material with an organic solvent.

The compounds of this invention have pharmacodynamic activity on the renal-cardiovascular system by means of a dopaminergic mechanism of action. Representative compounds have been demonstrated to increase renal blood flow and decrease renal vascular resistance in the anesthetized dog protocol which is a standard pharmacological procedure for measuring various renal and cardiovascular parameters. The prior art dopaminergic agent, dopamine, is used as a positive control. Details of this test system are available in U.S. Patent No. 4,197,297. Also, the compounds have demonstrated $D_2$-agonist activity in the isolated, perfused rabbit ear artery. Anti-hypertensive activity has, also, been demonstrated for the compounds of this invention in the spontaneously hypertensive rat (SHR) protocol.

Certain ester derivatives of the hydroxyl containing compounds of formula I, for example the 8-$C_{2-7}$-alkanoyl derivatives, act as prodrugs which give rise to the active parent compounds in vivo.

5-(2-Aminoethyl)-8-hydroxycarbostyril hydrochloride had an RVR-$ED_{15}$ of 1.8 µg/kg in the dog test. At 3 µg/kg/min by infusion in the renal dog screen, this compound decreased heart rate 22.5% and arterial blood pressure, −8%. Dopamine, as a standard, had an $ED_{15}$ of 3.5 µg/kg.

5-(2-Aminoethyl)-8-hydroxycarbostyril hydrochloride, also, lowered systolic blood pressure in the SHR at 25 mg/kg intraperitoneally administered.

5-(2-Di-*n*-propylaminoethyl)-8-hydroxycarbostyril, as the hemihydrate, increased renal blood flow in the dog protocol at 3 and 30 µg/kg/min infusion. It decreased systolic hypertension in the rat test at 12.5, 25 and 50 mg/kg, i.p.

5-[N-*n*--Propyl-N-(*p*-hydroxyphenethyl)aminoethyl)-8-hydroxycarbostyryl as the base had an $EC_{50}$ of 12nM in the isolated, perfused rabbit ear artery test protocol for evaluating presynaptic dopamine agonist or $D_2$ activity.

The compounds of formula I, therefore, are of utility in pharmaceutical agents for treating cardiovascular, renal, neurological or neoroendocrinological dysfunctions associated with dopamine deficiency. More specifically, the compounds are of use in treating hypertension or reduced kidney function.

The pharmaceutical compositions of this invention, which have pharmacodynamic activity, for example, renal vasodilation, correcting hemodynamic imbalance and antihypertensive activity, are incorporated, for administration, into conventional dosage unit forms. For example, one mixes a compound of formula I, or a pharmaceutically acceptable acid addition salt thereof, with a nontoxic pharmaceutical carrier, according to accepted pharmacy procedures, in a nontoxic quantity sufficient to produce the desired pharmacodynamic activity in an animal or human subject in need of such activity. The supplemented carrier is then converted into a dosage unit. Preferably, the compositions contain the active ingredient in an active but nontoxic quantity selected from the range of about 25 mg to about 400 mg, preferably about 75—200 mg, of active ingredient, as the base, per dosage unit.

The following examples are designed solely to illustrate the preparation and use of the compounds of this invention. The temperatures are Centigrade. Other variations of these examples will be obvious to those skilled in the art.

## Example 1

A mixture of 40 ml of 37% aqueous formaldehyde and 40 ml of concentrated hydrochloric acid was stirred while 36.5 g (.25 mol) of 8-hydroxyquinoline was added. Hydrogen chloride gas was bubbled through at a rate to maintain the temperature between 25—30°. After 2.5 hours, the reaction mixture was cooled to 5°. The separated solid was filtered and washed with ether to give 39.7 g (69%) of 5-(chloromethyl)-8-hydroxyquinoline; mp 275—280°.

A mixture of 44 g (.898 mol) of sodium cyanide and 1 liter of dimethylsulfoxide was stirred at 50° while 70 g (.3043 mol) of the chloromethyl compound was added portionwise over 10 minutes. The mixture was, then, stirred at 100° for 3 hours. It was quenched into 2 liters of ice/water, stirred 30 minutes and filtered to give 44.5 g (79%) of 5-cyanomethyl-8-hydroxyquinoline; mp 172—175°.

A mixture of 10 g (.0543 mol) of the cyanomethyl compound and 100 ml of dimethylformamide was treated with 2.9 g (.0597 mol) of 50% sodium hydride dispersion in mineral oil over a 20 minute period. The mixture was stirred at 50° for 2.5 hours, cooled to room temperature and 9.3 (.06516 mol) of methyl iodide in 10 ml of dimethylformamide was added dropwise over 10 minutes. The mixture was stirred at 50° for 30 minutes, then, poured onto 70 ml of ice/water. The mixture was extracted four times with ethyl acetate. The extract was dried, evaporated and the residue triturated with ether, followed by isopropyl alcohol, to give 5 g (46%) of 5-cyanomethyl-8-methoxyquinoline; mp 147—151°.

A mixture of 14.5 g (.0732 mol) of the cyano compound, 300 ml of methanolic ammonia and 4 g of Raney nickel was hydrogenated at 344X$10^3$ Pa of hydrogen for 3 hours until the theoretical hydrogen

uptake was complete. The reaction mixture was filtered, evaporated and stirred with 100 ml of acetic anhydride at room temperature for 10 minutes. It was stripped from water three times and the residue dried in chloroform. The solvent was stripped and the residue was recrystallized from acetone/ether to give 12 g (63%) of 5-acetamidoethyl-8-methoxyquinoline; mp 155—156°.

Anal. Calcd. for $C_{14}H_{16}N_2O_2 \cdot 1/4\ H_2O$: C, 67.59; H, 6.69; N, 11.26. Found: C, 67.95; H, 6.57; N, 10.93.

A mixture of 12 g (.0492 mol) of the above quinoline and 200 ml of chloroform was treated with 15.5 g of 80% *m*-chloroperbenzoic acid at room temperature overnight. The reaction mixture was stripped and the residue dissolved in chloroform, then, filtered from alumina twice. The mother liquor was evaporated and residue was recrystallized from acetone/ether to give 6.5 g (51%) of the N-oxide; mp 147—148°.

Anal. Calcd. for $C_{14}H_{16}N_2O_3 \cdot 3/8\ H_2O$: C, 62.96; H, 6.32; N, 10.48. Found: C, 62.88; H, 6.14; N, 10.30.

A mixture of 4 g (.0154 mol) of the N-oxide in 50 ml of acetic anhydride was heated at 100° for 30 minutes, stripped from water twice and dried in chloroform. The volatiles were stripped, the residue passed over a dry alumina column, using 1:1 chloroform/hexane to 10:1 methanol/chloroform, to give an oil which was recrystallized from acetone to give 1.5 g (37.5%) of 5-acetamidoethyl-8-methoxycarbostyril; mp 168—169°.

Anal. Calcd. for $C_{14}H_{16}N_2O_3 \cdot 1/4\ H_2O$: C, 63.50; H, 6.28; N, 10.58. Found: C, 63.27; H, 6.57; N, 10.27.

A mixture of 1.8 g (.00692 mol) of the carbostyril and 200 ml of methylene chloride at 0° was treated with 24 ml of boron tribromide solution (1 g/2.5 ml of methylene chloride). The mixture was stirred for 12 hours at room temperature and was, then, quenched with methanol. The volatiles were stripped several times. The residue was taken through methanol, then, triturated with acetone to give 2.5 g of crude 8-hydroxy product (TLC silica, 20% methanol/chloroform: Rf sm = 0.6; p = 0.4).

The crude material was refluxed for 6 hours in 6N hydrochloric acid and stripped. The residue was recrystallized from water/isopropanol to give .750 g (45%) of 5-aminoethyl-8-hydroxycarbostyril hydrochloride; mp 310—311° (d) (water loss at 110°).

Anal. Calcd. for $C_{14}H_{16}N_2O_2 \cdot HCl \cdot H_2O$: C, 51.07; H, 5.84; N, 10.83. Found: C, 51.15; H, 5.65; N, 10.95.

A small sample was dried to give the anhydrous form.

Anal. Calcd. for $C_{11}H_{12}N_2O_2$: C, 54.89; H, 5.44; N, 11.64. Found: C, 54.20; H, 5.70; N, 11.68.

## Example 2

A mixture of 20 g (.1010 mol) of 5-cyanomethyl-8-hydroxyquinoline and 300 ml of methanolic ammonia was hydrogenated at $344X10^3$ Pa of hydrogen with 4 g of Raney nickel for 4 hours until the theoretical quantity of hydrogen uptake was complete. The mixture was filtered, the filtrate evaporated and the residue dissolved in 300 ml of chloroform. The dried extract was treated with 14 ml (.101 mol) of triethylamine and 11.7 ml (.101 mol) of benzoyl chloride. The mixture was poured onto ice/water, basified with ammonium hydroxide and extracted 3 times by chloroform. The extracts were washed with water, back washed with chloroform, dried and evaporated. The residue was crystallized from acetone/ether to give 20 g (65%) 5-benzoylaminoethyl-8-methoxyquinoline; mp 143—147°.

A mixture of 22 g (.0719 mol) of the benzoylamino compound and 200 ml of chloroform was stirred overnight at room temperature with 24 g of (80%) *m*-chloroperbenzoic acid. The mixture was poured into 700 ml of 5% sodium bicarbonate solution, then was extracted 6 times with chloroform and passed quickly through dry alumina inh 10% methanol/chloroform. The solution was evaporated. The residue was crystallized from acetone/ethyl acetate to give 15 g (65%) of N-oxide; mp 128—130°.

The crude N-oxide (.06536 mol) was dissolved in 400 ml of tetrahydrofuran at 0°, and treated with 45 ml (.323 mol) of triethylamine and 65 ml (.6889 mol) of acetic anhydride. After 10 minutes, the mixture was poured into 1400 ml of 5% bicarbonate, then, extracted 5 times with ethyl acetate. The dried extract was evaporated and the residue triturated with acetone to give 12 g (57%) of 5-benzoylaminoethyl-8-methoxycarbostyril; mp 155—158°.

A mixture of 4.5 g (.01398 mol) of this carbostyril, 100 ml of methanol and 100 ml of 10% sodium hydroxide was refluxed for 24 hours until starting material was no longer present by thin layer analysis (Rf sm = .9; p = .2; 20:1 methanol/chloroform over silica). The mixture was acidified with concentrated hydrochloric acid, washed twice with ethyl acetate, then, basified with ammonium hydroxide and continuously extracted with chloroform. The chloroform was evaporated and the residual oil taken into 200 ml of acetic acid. The mixture was hydrogenated at $344X10^3$ Pa of hydrogen with 14 ml (.194 mol) of propanal and 10% palladium-on-charcoal for 2 hours. The mixture was filtered, the filtrate evaporated, diluted with water and basified with ammonium hydroxide. The mixture was extracted twice with ethyl acetate. The extract was dried, evaporated and the residue chromatographed over silica (1:1 hexane/chloroform→chloroform→10% methanol/chloroform) to give 3 g of oily base (71%). After reacting with fumaric acid, the recovered fumarate salt of 5-di-*n*-propylamino-8-methoxycarbostyril was recrystallized from methanol/ethyl acetate/ether; mp 161.5—162°.

Anal. Calcd. for $C_{18}H_{26}N_2O_2 \cdot 1/2\ H_2O$: C, 61.81; H, 7.31; N, 6.55. Found: C, 61.88; H, 7.22; N, 6.02.

The 5-methoxycarbostyril, 2.7 g (.00894 mol), was dissolved in 50 ml of methylene chloride and treated with 20 ml (1 g/2.5 ml methylene chloride) of boron tribromide solution. The mixture was stirred 1/2 hour at room temperature, then, refluxed 10 minutes.

The reaction mixture was quenched with methanol and stripped. The residue was diluted with water and washed twice with ethyl acetate. The aqueous layer was, then, brought to pH 8 with ammonium

hydroxide and extracted seven times with ethyl acetate. The extracts were dried and evaporated. The extract residue was suspended in 50 ml of boiling water. After cooling 1 hour in the refrigerator, 1.7 g (66%) of 5-di-*n*-propylaminoethyl-8-hydroxycarbostyril was collected; mp 228—230°.

Anal. Calcd. for $C_{17}H_{24}N_2O_2$: C, 70.80; H, 8.39; N, 9.71. Found: C, 71.20; H, 8.69; N, 9.44.

The base (500 mg) is dissolved in ether/ethyl acetate and reacted with hydrogen bromide gas to give the hydrobromide salt. The hydrochloride salt is prepared similarly. Another portion (500 mg) is reacted with an excess of methanesulfonic acid to give the acid addition salt.

Example 3

5-Benzoylaminoethyl-8-methoxycarbostyril from above, 12 g (.0373 mol), was refluxed for 24 hours in 100 ml of methanol and 100 ml of 10 N sodium hydroxide. The reaction mixture was poured onto ice/water, acidified with concentrated hydrochloric acid, washed twice with ether, basified with ammonium hydroxide and continuously extracted with chloroform to give 12 g of crude oil.

The oil was treated with 13.4 ml (.1318 mol) of benzaldehyde and azeotroped in toluene (~200 ml) for 2 hours or until the starting material was gone by TLC, (silica 20%, methanol/chloroform: Rf sm = .2; p = .5). The toluene was evaporated. The residual oil dissolved in 100 ml of methanol at 0° and treated with 7 g (.1852 mol) of sodium borohydride, portionwise. After 1/2 hour at 0°, the reaction was complete. (TLC, silica 10%, methanol/chloroform: Rf sm = .5; p = .4).

The mixture was poured into ice/water, treated with ammonium hydroxide and extracted three times with chloroform. The extracts were washed with water, dried and evaporated. The residue was dissolved in methanol and acidified with ethereal hydrogen chloride. Recrystallization from methanol/ethyl acetate/ether gave 5-benzylaminoethyl-8-methoxycarbostyril hydrochloride; mp 266—268°. The free base was liberated using ammonium hydroxide and extracted with ethyl acetate (11 g) to give clean base (oil) (96%).

The benzyl carbostyril, 12 g (.0389 mol), was refluxed 1/2 hour in 100 ml of dimethylformamide, 15 ml (.1098 mol) of phenethyl bromide and 50 g (.362 mol) of potassium carbonate. The mixture was poured onto 1 liter of ice/water. The quench was extracted three times with ethyl acetate. The extracts were washed four times with water, dried and evaporated to give 2 major spots by TLC (10% methanol/chloroform, silica: Rf sm = .4; p = .9, .8).

The oil was dissolved in 100 ml of methanol, acidified to pH 1 with hydrochloric acid and hydrogenated with 3 g of 10% palladium-on-charcoal until no starting material remained by TLC. The mixture was filtered, stripped, basified with ammonium, and extracted twice with ethyl acetate. The extracts were dried, evaporated, and the residue recrystallized from ether to give 2.5 g (20%) 5-phenethylaminoethyl-8-methoxycarbostyril; mp 92—93° (TLC, 10% methanol/chloroform, silica: Rf = .5).

Anal. Calcd. for $C_{20}H_{22}N_2O_2$: C, 74.51; H, 6.88; N, 8.69. Found: C, 73.95; H, 6.81; N, 8.52.

The mother liquor was evaporated and chromatographed over silica (7:3 ethyl acetate/hexane→ethyl acetate) to give 3.5 g (21%) of N-phenethyl-O-phenethyl by-product (Rf = .6; 10:1 methanol/chloroform, silica).

This was converted to the hydrochloride salt; mp 142—144°.

Anal. Calcd. for $C_{28}H_{30}N_2O_2 \cdot HCl$: C, 67.33; H, 6.46; N, 5.61. Found: C, 67.36; H, 6.61; N, 5.59.

The by-product was converted to 5-phenethylaminoethyl-8-hydroxycarbostyril hydrochloride (1.5 g) by refluxing in 8:2 hydrochloric acid:water (50 ml).

Example 4

5-(Phenethylaminoethyl)-8-methoxycarbostyril from above, 2.5 g (.00776 mol), was dissolved in 100 ml of glacial acetic acid, treated with 2.5 ml (.0347 mol) of propanol and hydrogenated at $344 \times 10^3$ Pa of hydrogen with 2 g of 10% palladium-on-charcoal for 1/2 hour (TLC, 10% methanol/chloroform, silica; Rf sm = .5; p = .7). The mixture was filtered, stripped, diluted with water, basified with ammonium hydroxide and extracted twice with ethyl acetate. The extracts were washed with water, dried and evaporated to give 2.5 g oil (89%); 5-(N-*n*-propyl-N-phenethylaminoethyl)-8-methoxycarbostyril.

The methoxycarbostyril, 2.2 g (.00604 mol), was dissolved in 100 ml of methylene chloride at room temperature and treated with 20 ml of 1M boron tribromide (.02 mol). The mixture was refluxed for 1 hour, (TLC, 10% methanol/chloroform, silica: Rf sm = .7; p = .6). It was quenched and stripped from methanol, slurried in water, brought to pH 8 with ammonium hydroxide and extracted twice with ethyl acetate. The extracts were washed with water, dried, evaporated and the residue triturated with ether. The solid was recrystallized from hot methanol/water (1:1) to give 1.3 g (62%) of 5-(N-*n*-propyl-N-phenethylaminoethyl)-8-hydroxycarbostyril, mp 183—184°.

Anal. Calcd. for $C_{22}H_{26}N_2O_2$: C, 75.40; H, 7.48; N, 7.99. Found: C, 75.18; H, 7.36; N, 7.84.

Example 5

*p*-Methoxybenzaldehyde (7.3 ml, .0602 mol) was stirred at room temperature in 65 ml of methylene chloride, 25 ml of 40% sodium hydroxide, .144 g of benzyl, triethylammonium chloride and 4.2 ml of chloroacetonitrile for 1/2 hour. The reaction mixture was extracted twice with methylene chloride. The extracts were washed with 5% sodium bisulfite, water, dried and evaporated to give 8.1 g of the desired cyanoepoxide (oil) (77%).

The epoxide (7.8 g, .0446 mol) was refluxed 1/2 hour in 50 ml of water with .032 ml of sulfuric acid.

Crude 5-(2-aminoethyl-8-methoxycarbostyril (8.1 g, .0372 mol) dissolved in 40 ml of water was added. The mixture was immediately poured into water and extracted twice with ethyl acetate. The extracts were dried, evaporated and triturated with ethyl acetate to give 11 g (75%) α-cyano-β-hydroxy product (TLC, silica, 10% methanol/chloride; Rf sm = .2, p = .3); mp = 135—136°.

The α-cyano product from above (11 g, .02798 mol) was suspended in 30 ml of ethanol and treated with 28 g of sodium borohydride (.7407 mol) at room temperature for 24 hours. An unknown by-product of faster Rf (TLC, 20% methanol/chloride; sm = .6, p = .4, unknown = .5) was present. The reaction mixture was quenched with acetic acid and water, basified with ammonium hydroxide and extracted four times with ethyl acetate. The dried extracts were evaporated and the residue triturated with ethyl acetate to give 5 g (50%) of 5-[N-2-(*p*-methoxyphenyl)-2-hydroxy-ethylamino]-8-methoxycarbostyril; mp 157—162°.

The β-hydroxy product from above (1.3 g, .00353 mol) was stirred for 10 minutes with 50 ml of hydrochloric acid and 50 ml of acetic acid at 100°, then, hydrogenated directly with 1 g of 10% palladium-on-charcoal at $344X10^3$ Pa of hydrogen. After 1 hour, the mixture was filtered, diluted with water, basified with ammonia and extracted twice with ethyl acetate. The extracts were dried and evaporated to give 1.2 g of 5-(N-*p*-methoxyphenethylaminoethyl)-8-methoxycarbostyril; mp 89—91°.

This secondary amine (1 g, .00284 mol) was alkylated by hydrogenation at $344X10^3$ Pa of hydrogen with 1 g of 10% palladium-on-charcoal, 100 ml of acetic acid and 3 ml (.042 mol) of propanal for 1/2 hour. The mixture was filtered, evaporated, diluted with water/ammonium hydroxide and extracted twice with ethyl acetate. The extracts were dried and evaporated to give .95 g of 5-(N-*n*--propyl-N-(*p*-methoxyphenethyl)aminoethyl)-8-methoxycarbostyril as a crude oil (86%). This material (500 mg) in ethyl acetate/ether is saturated with hydrogen chloride gas to give the hydrochloride salt.

The methoxy compound (.95 g, .0024 mol) was dissolved in 50 ml of methylene chloride and treated with 15 ml of boron tribromide solution (1 g/2.5 ml methylene chloride) at room temperature. The mixture was refluxed for 1 hour, quenched with methanol, evaporated, diluted with water, brought to pH 8 with ammonia and extracted twice with ethyl acetate. The extract was dried and evaporated. The residue was triturated with ether and suspended in hot methanol/water (1:2). The suspension was cooled in the refrigerator overnight, then, filtered. The solid was recrystallized from acetonitrile to give .2 g (23%) of 5-[N-*n*-propyl-N-(*p*-hydroxyphenethyl)aminoethyl)-8-hydroxycarbostyril; mp 176—178°C.

Example 6

5-[N-(*p*-methoxyphenethyl)aminoethyl]-8-methoxycarbostyril (0.55 g) was dissolved in 50 ml of methylene chloride at −10°C, then, treated with 15 ml of boron tribromide-methylene chloride (1 g:12.5 ml). The mixture was stirred at room temperature for one-half hour and heated at reflux for one hour. After quenching in methanol, the methanol was concentrated to give, after recrystallization from ethanol-ethyl acetate, 0.158 g of 5-[N-(*p*-hydroxyphenethyl)aminoethyl]-8-hydroxycarbostyril hydrobromide, m.p. 248—250°.

Anal. Calcd. for $C_{19}H_{20}N_2O_3 \cdot HBr \cdot 3/4\ H_2O$: C, 54.49; H, 5.42; N, 6.69. Found: C, 54.59; H, 5.32; N, 6.78.

**Claims for the Contracting States: BE CH DE FR GB IT LI LU NL SE**

1. A compound of the structural formula:

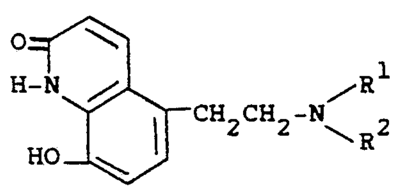

I

in which $R_1$ and $R_2$ are, each, hydrogen, a straight or branched $C_{1-6}$ alkyl group, allyl, benzyl, phenethyl or *p*-hydroxyphenethyl, a pharmaceutically acceptable, acid addition salt or an 8-$C_{2-7}$-alkanoyl derivative thereof.

2. The compound according to claim 1 in which —$NR^1R^2$ is amino, di-*n*-propylamino, *n*-propyl-*n*-butylamino or *p*-hydroxyphenethyl-*n*-propylamino.

3. The compound according to claim 1 which is 5-[N-*n*-propyl-N-(*p*-hydroxyphenethyl)aminoethyl]-8-hydroxycarbostyril, a salt or an 8-$C_{2-7}$-alkanoyl derivative thereof.

4. The compound according to claim 1 which is 5-(di-*n*-propylaminoethyl)-8-hydroxycarbostyril.

5. The compound according to claim 1 which is 5-(di-*n*-propylaminoethyl)-8-hydroxycarbostyril hydrochloride.

6. The compound according to claim 1 which is 5-aminoethyl-8-hydroxycarbostyril.

7. A compound according to any of claims 1—6 for pharmaceutical use.

8. The process for preparing a compound of claim 1, which comprises deprotecting a compound of the formula:

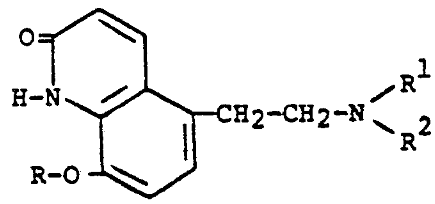

II

in which $R^1$ and $R^2$ are as defined in claim 1 and R is a protecting group and, optionally, forming a salt or an 8-$C_{2-7}$-alkanoyl derivative.

9. The process of claim 8 in which R is methyl and the process is run using boron tribromide as the deprotecting agent.

10. The process of claim 8 in which R is methyl and the process is run using 48% aqueous hydrogen bromide as the deprotecting agent.

**Claims for the Contracting State: AT**

1. The process for preparing a compound of the formula:

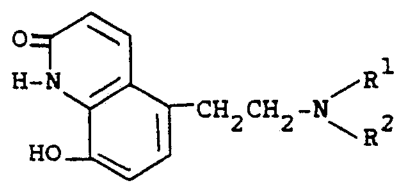

I

in which $R_1$ and $R_2$ are, each, hydrogen, a straight or branched $C_{1-6}$ alkyl group, allyl, benzyl, phenethyl or *p*-hydroxyphenethyl, a pharmaceutically acceptable salt thereof, which comprises deprotecting a compound of the formula:

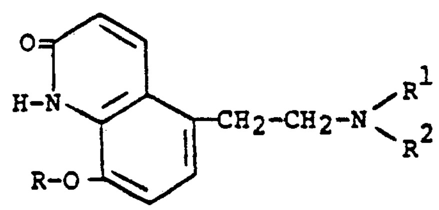

II

in which R is a protecting group and $R_1$ and $R_2$ are as defined above, and, optionally, forming a salt or an 8-$C_{2-7}$-alkanoyl derivative.

2. The process of claim 1 in which R is methyl and the process is run using boron tribromide as the deprotecting agent.

3. The process of claim 1 in which R is methyl and the process is run using 48% aqueous hydrogen bromide as the deprotecting agent.

4. The process of claim 1 in which R is methyl and the product is 5-[N-*n*-propyl-N-(*p*-hydroxyphenethyl)aminoethyl]-8-hydroxycarbostyril.

5. The process of claim 1 in which R is methyl and the product is 5-aminoethyl-8-hydroxycarbostyril.

**Patentansprüche für die Vertragsstaaten: BE CH DE FR GB IT LI LU NL SE**

1. Eine Verbindung der Strukturformel:

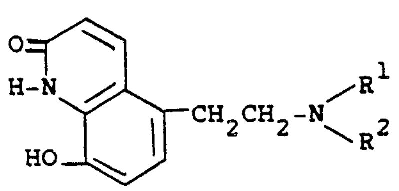

I

in der $R^1$ und $R^2$ jeweils ein Wasserstoffatom, einen geradkettigen oder verzweigten $C_{1-6}$-Alkylrest, eine Allyl-, Benzyl-, Phenäthyl- oder p-Hydroxyphenäthylgruppe bedeuten, ein pharmazeutisch verträgliches Säureadditionssalz oder ein 8-$C_{2-7}$-Alkanoylderivat davon.

2. Verbindung nach Anspruch 1, in der $NR^1R^2$ eine Amino-, Di-n-propylamino-, n-Propyl-n-butylamino- oder p-Hydroxyphenäthyl-n-propylaminogruppe bedeutet.

3. Verbindung nach Anspruch 1, nämlich 5-[N-n-Propyl-N-(p-hydroxyphenäthyl)-aminoäthyl]-8-hydroxycarbostyril, ein Salz oder ein 8-$C_{2-7}$-Alkanoylderivat davon.

4. Verbindung nach Anspruch 1, nämlich 5-(Di-n-propylaminoäthyl)-8-hydroxycarbostyril.

5. Verbindung nach Anspruch 1, nämlich 5-(D-n-propylaminoäthyl)-8-hydroxycarbostyril-hydrochlorid.

6. Verbindung nach Anspruch 1, nämlich 5-Aminoäthyl-8-hydroxycarbostyril.

7. Eine Verbindung nach einem der Ansprüche 1 bis 6 zur pharmazeutischen Verwendung.

8. Verfahren zur Herstellung einer Verbindung nach Anspruch 1, das Abspaltung der Schutzgruppe von einer Verbindung der Formel:

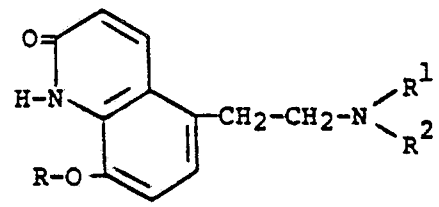

II

in der $R^1$ und $R^2$ die in Anspruch 1 angegebene Bedeutung haben und R eine Schutzgruppe ist, und, gegebenenfalls Erzeugung eines Salzes oder eines 8-$C_{2-7}$-Alkanoylderivates davon umfaßt.

9. Verfahren nach Anspruch 8, wobei R eine Methylgruppe bedeutet und das Verfahren unter Verwendung von Bortribromid als Mittel zur Entfernung der Schutzgruppe durchgeführt wird.

10. Verfahren nach Anspruch 8, wobei R eine Methylgruppe bedeutet und das Verfahren mit 48%igem wäßrigem Brom-wasserstoff als Mittel zur Entfernung der Schutzgruppe durchgeführt wird.

**Patentansprüche für den Vertragstaat: AT**

1. Ein Verfahren zur Herstellung einer Verbindung der Strukturformel:

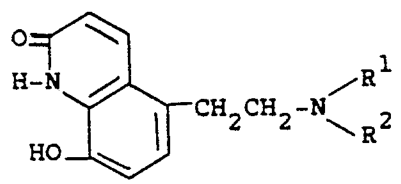

I

in der $R^1$ und $R^2$ jeweils ein Wasserstoffatom, einen geradkettigen oder verzweigten $C_{1-6}$-Alkylrest, eine Allyl-, Benzyl-, Phenäthyl- oder p-Hydroxyphenäthylgruppe bedeuten oder eines pharmazeutisch verträglichen Salzes davon, das Abspaltung der Schutzgruppe von einer Verbindung der Formel:

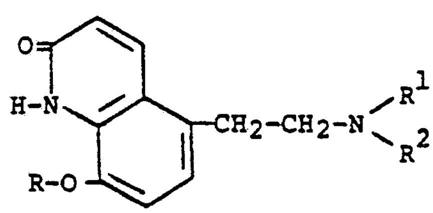

II

in der R eine Schutzgruppe ist und $R^1$ und $R^2$ die vorstehend angegebene Bedeutung haben und, gegebenenfalls, Erzeugung eines Salzes oder eines 8-$C_{2-7}$-Alkanoylderivates davon umfaßt.

2. Verfahren nach Anspruch 1, wobei R eine Methylgruppe bedeutet und das Verfahren unter Verwendung von Bortribromid als Mittel zur Entfernung der Schutzgruppe durchgeführt wird.

3. Verfahren nach Anspruch 1, wobei R eine Methylgruppe bedeutet und das Verfahren mit 48%igem wäßrigem Bromwasserstoff als Mittel zur Entfernung der Schutzgruppe durchgeführt wird.

4. Verfahren nach Anspruch 1, wobei R eine Methylgruppe bedeutet, und 5-[N-n-Propyl-N-(p-hydroxyphenäthyl)-aminoäthyl]-8-hydroxycarbostyril hergestellt wird.

5. Das Verfahren nach Anspruch 1, wobei R eine Methylgruppe bedeutet und 5-Aminoäthyl-8-hydroxycarbostyril hergestellt wird.

**Revendications pour les Etats Contractants: BE CH DE FR GB IT LI LU NL SE**

1. Un composé de la formule structurelle suivante:

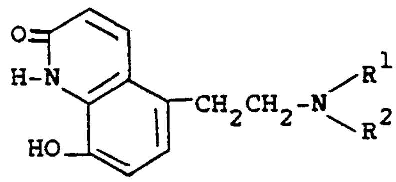

I

dans laquelle $R^1$ et $R^2$ sont chacun de l'hydrogène, un groupe alcoyle $C_{1-6}$ droit ou ramifié, allyle, benzyle, phénétyle ou *p*-hydroxyphénéthyle, un sel d'addition d'acide, pharmaceutiquement acceptable ou un dérivé 8-$C_{2-7}$-alcanoyl du même.

2. Le composé selon la revendication 1 dans lequel —$NR^1R^2$ est amino, di-*n*-propylamino, *n*-propyle-*n*-butylamino ou *p*-hydroxyphénéthyle-*n*-propylamino.

3. Le composé selon la revendciation 1 qui est 5-[N-*n*-propyle-N-(*p*-hydroxyphénéthyle)aminoéthyle]-8-hydroxycarbostyrile, un sel ou un dérivé 8-$C_{2-7}$-alcanoyl du même.

4. Le composé selon la revendication 1 qui est 5-(di-*n*-propylaminoéthyle)-8-hydroxycarbostyrile.

5. Le composé selon la revendication 1 qui est le chlorhydrate 5-(di-*n*-propylaminoéthyle)-8-hydroxycarbostyrile.

6. Le composé selon la revendication 1 qui est 5-aminoéthyle-8-hydroxycarbostyrile.

7. Un composé selon n'importe laquelle des revendication de 1 à 6 à usage pharmaceutique.

8. Le procédé pour la préparation d'un composé de la revendication 1 qui comprend le retrait de la protection d'un composé de la formule:

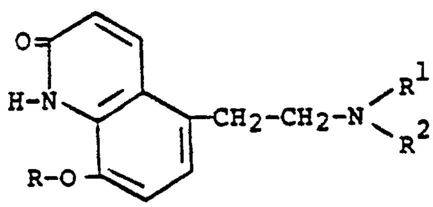

II

dans laquelle $R^1$ et $R^2$ sont comme définis dans la revendication 1 et R est un groupe protecteur et, facultativement, la formation d'un sel ou d'un dérivé 8-$C_{2-7}$-alcanoyl.

9. Le procédé de la revendication 8 dans lequel R est du méthyle et le procédé est réalisé en se servant de tribromure de bore en tant qu'agent de retrait de protection.

10. Le procédé de la revendication 8 dans lequel R est du méthyle et le procédé est effectué en se servant de 48% d'acide bromhydrique aqueux en tant qu'agent de retrait de protection.

**Revendications pour l'Etat contractant: AT**

1. Le procédé de préparation d'un composé de la formule:

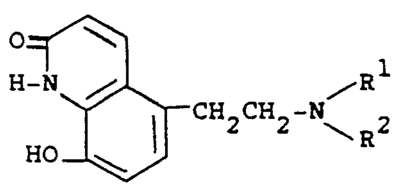

I

dans lequel $R^1$ et $R^2$ sont chacun, hydrogène, un groupe alcoyle $C_{1-6}$ droit ou ramifié, allyle, benzyle, phénéthyle ou *p*-hydroxyphénéthyle, un sel du même pharmaceutiquement acceptable, qui comprend le retrait de la protection d'un compose de la formule:

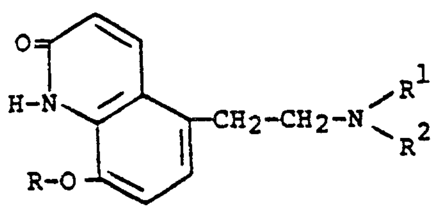

II

dans lequel R est un groupe de protection et $R^1$ et $R^2$ sont comme définis ci-dessus, et, facultativement, la formation d'un sel ou d'un dérivé 8-alcanoyl $C_{2-7}$.

2. Le procédé de la revendication 1 dans lequel R est du méthyle et le procédé est réalisé en se servant de tribromure de bore en tant qu'agent de retrait de protection.

3. Le procédé de la revendication 1 dans lequel R est du méthyle et le procédé est effectué en se servant de 48% d'acide bromhydrique aqueux en tant qu'agent de retrait de protection.

4. Le procédé de la revendication 1 dans lequel R est du méthyle et le produit est 5-[N-*n*-propyle-N-(*p*-hydroxyphénéthyle)aminoéthyle]-8-hydroxycarbostyrile.

5. Le procédé de la revendication 1 dans lequel R est du méthyle et le produit est 5-aminoéthyle-8-hydroxycarbostyrile.